# EUROPÄISCHE PATENTSCHRIFT

(11) **EP 2 786 126 B1**
(45) Veröffentlichungstag und Bekanntmachung des Hinweises auf die Patenterteilung: **11.10.2017**
(21) Anmeldenummer: 12819055.0
(22) Anmeldetag: 30.11.2012
(51) Int. Cl.: G01N 33/18, G01N 21/33, G01N 21/3577, G01N 21/359, G01N 21/64

(54) **VERFAHREN UND VORRICHTUNG ZUR DETEKTION VON FREMDSTOFFEN IN WASSER**
METHOD AND DEVICE FOR DETECTING FOREIGN SUBSTANCES IN WATER
PROCÉDÉ ET DISPOSITIF DESTINÉS À DÉTECTER DES CONTAMINANTS DANS L'EAU

(30) Priorität: 02.12.2011 DE 102011087673
(43) Veröffentlichungstag der Anmeldung: 08.10.2014
(73) Patentinhaber: Unisensor Sensorsysteme GmbH, 76149 Karlsruhe (DE)
(72) Erfinder: KRIEG, Gunther, 76227 Karlsruhe (DE); FEY, Dirk, F-67630 Neewiller (FR); MERTINEIT, Sabine, 76287 Rheinstetten (DE); HAPPEL, Oliver, 76199 Karlsruhe (DE)
(74) Vertreter: Patent- und Rechtsanwälte Ullrich & Naumann
(86) Internationale Anmeldenummer: PCT/DE2012/200079
(87) Internationale Veröffentlichungsnummer: WO 2013/079064

(56) Entgegenhaltungen:
- EP-A2- 0 647 848
- DE-C1- 3 931 360
- US-A- 3 287 088
- US-A- 5 460 973
- AOIFE A. GOWEN: "Investigation of the Potential of Near Infrared Spectroscopy for the Detection and Quantification of Pesticides in Aqueous Solution", AMERICAN JOURNAL OF ANALYTICAL CHEMISTRY, Bd. 02, Nr. 08, 1. Januar 2011 (2011-01-01), Seiten 53-62, XP55056563, ISSN: 2156-8251, DOI: 10.4236/ajac.2011.228124
- HORVAT M ET AL: "Comparison of distillation with other current isolation methods for the determination of methyl mercury compounds in low level environmental samples", ANALYTICA CHIMICA ACTA, ELSEVIER, AMSTERDAM, NL, Bd. 282, Nr. 1, 5. Oktober 1993 (1993-10-05), Seiten 153-168, XP026694865, ISSN: 0003-2670, DOI: 10.1016/0003-2670(93)80364-Q [gefunden am 1993-10-05]
- F. REGAN ET AL: "Determination of pesticides in water using ATR-FTIR spectroscopy on PVC/chloroparaffin coatings", ANALYTICA CHIMICA ACTA, Bd. 334, Nr. 1-2, 1. November 1996 (1996-11-01), Seiten 85-92, XP055056559, ISSN: 0003-2670, DOI: 10.1016/S0003-2670(96)00259-0

## Beschreibung

Die Erfindung betrifft ein Verfahren zur Detektion von Fremdstoffen, insbesondere von Schadstoffen, in Wasser.

Die Erfindung betrifft ebenfalls eine Vorrichtung zur Detektion von Fremdstoffen, insbesondere von Schadstoffen, in Wasser.

Obwohl auf beliebige Wässer anwendbar, wird die vorliegende Erfindung in Bezug Trinkwasser beschrieben.

Als Fremdstoffe im Sinne der Erfindung sind insbesondere in der Beschreibung, vorzugsweise in den Ansprüchen endokrine Wirkstoffe, Pflanzenschutzmittel, Körperpflegestoffe, und/oder Industriechemikalien zu verstehen. Unter dem Begriff Pflanzenschutzmittel sind insbesondere in der Beschreibung, vorzugsweise in den Ansprüchen Herbizide, Fungizide und/oder Insektizide zu verstehen. Unter dem Begriff endokriner Wirkstoff sind insbesondere in der Beschreibung, vorzugsweise in den Ansprüchen, Hormone zu verstehen.

Unter Wasser ist insbesondere in der Beschreibung, vorzugsweise in den Ansprüchen Oberflächenwasser, Flusswasser, Trinkwasser oder Abwasser zu verstehen. Schadstoffe im Sinne der Erfindung sind insbesondere in der Beschreibung, vorzugsweise in den Ansprüchen, Stoffe oder Stoffgemische, die schädlich für Menschen, Tiere und/oder Pflanzen sind. Hierunter fallen insbesondere natürliche und/oder künstliche Schadstoffe.

Trinkwasser ist eines der wichtigsten Lebensmittel. Der Qualitätskontrolle von Trinkwasser kommt daher eine hohe Relevanz zu. So wird beispielsweise von den Wasserversorgern erwartet, dass jederzeit einwandfreies Trinkwasser an Endverbraucher, wie beispielsweise Bürger oder Firmen abgegeben wird. In der Getränkeindustrie wird Trinkwasser unter anderem zum Verdünnen von Konzentraten, wie Fruchtsaftkonzentraten oder Limonadensirups eingesetzt und ist dann Be-Bestandteil des fertigen Produktes. Zur regelmäßigen Qualitätskontrolle der Trinkwasserqualität sind Systeme bekannt geworden, die beispielsweise eine Leitfähigkeit, einen pH-Wert, eine Trübung, einen SAK₂₅₄-Wert, eine Temperatur, einen Sauerstoffgehalt oder ein Ozon- und/oder Chlorgehalt des Trinkwassers messen können. Die so erhaltenen Werte können dann zur Regelung der Trinkwasseraufbereitung und/oder Betriebsüberwachung herangezogen werden.

Um komplexere Verbindungen im Trinkwasser feststellen zu können, sind selektive Analysetechniken bekannt geworden: Um organische Verbindungen nachweisen zu können, sind beispielsweise gaschromatographische oder flüssigkeitschromatographische Methoden bekannt geworden. Um Metalle nachweisen zu können kann Massenspektrometrie mit induktiv gekoppeltem Plasma (ICP-MS) verwendet werden. Diese genannten Methoden erlauben zwar eine spezifische Detektion und Quantifizierung der genannten Fremd- und Schadstoffe mit gewünschter Genauigkeit. Nachteilig ist jedoch, dass diese sowohl einen großen personellen und instrumentellen Aufwand erfordern. Darüber hinaus ist auch der zeitliche Aufwand hoch. So können aufgrund des entsprechenden Aufwandes und der damit verbundenen Kosten nur längerfristige Trends in dem Trinkstoffgehalt im Trinkwasser aufgedeckt werden. Bei einer unentdeckten Leckage, beispielsweise von Betriebsstoffen, wie Öl, etc. ins Trinkwasser, kann dieses lange Zeit unbemerkt bleiben, obwohl eine eventuell kritische Kontamination des Trinkwassers mit dem jeweiligen Fremdstoff auftritt.

In der US 5,460,973 ist ein Verfahren zum optischen Messen von flüchtigen Fremdstoffen in einem damit kontaminierten Medium gezeigt. Dabei wird das zu analysierende Medium zunächst erhitzt und dann in Richtung auf ein Sensorelement versprüht. Das Sensorelement ist mit einer Membran umgeben, die selektiv einen vorgegebenen Analyten absorbiert und Wasser ausschließt. Anschließend wird im Infrarotbereich die Absorption detektiert, in dem beispielsweise mit einer gewöhnlichen Infrarotquelle in ein Totalreflexionselement eingestrahlt wird, welches mit besagter Membran in Kontakt steht.

In der DE 39 31 360 C1 ist ein Verfahren zur Bestimmung geringer Mengen von Herbiziden auf der Basis von Triazinverbindungen anhand einer Flüssig-Flüssig-Extraktoren gezeigt.

Aus der Nichtpatentliteratur AOIFE A. GOWEN: "Investigation of the Potential of Near Infrared Spectroscopy for the Detection and Quantification of Pesticides in Aqueous Solution", American Journal of Analytical Chemistry, Bd. 02, Nr. 08, 1. Januar 2011 (2011-01-01), Seiten 53-62, XP055056563, ISSN: 2156-8251, DOI: 10.4236/ajac.2011.228124 ist ein Verfahren zur Bestimmung von Herbiziden in wässrigen Lösungen anhand von NIR-Spektroskopie bekannt geworden.

Aus der Nichtpatentliteratur HORVAT M et al.: "Comparison of distillation with other current isolation methods for the determination of methyl mercury compounds in low level environment samples", Analytica Chimica Acta, Elsevier, Amsterdam, NL, Bd. 282, Nr. 1, 5. Oktber 1993 (1993-10-05), Seiten 153-168, XP026694865, ISSN: 0003-2670, DOI: 10.1016/0003-2670(93)80364-Q ist ein Verfahren zur Bestimmung von Methylquecksilber bekannt geworden.

Eine Aufgabe der vorliegenden Erfindung ist es daher, ein Verfahren und eine Vorrichtung anzugeben, welche eine kostengünstige und kontinuierliche Überwachung von Fremdstoffen in Wasser ermöglicht. Eine weitere Aufgabe der vorliegenden Erfindung ist es, ein Verfahren und eine Vorrichtung anzugeben, welche den personellen und instrumentellen Aufwand senkt und gleichzeitig zuverlässig ist. Eine weitere Aufgabe der vorliegenden Erfindung ist schließlich, eine Vorrichtung und ein Verfahren anzugeben, welches eine Vielzahl von verschiedenen Fremdstoffen in Wasser mit ausreichender Genauigkeit erkennt.

Die vorliegende Erfindung löst die Aufgaben durch ein Verfahren zur Detektion von Fremdstoffen in Wasser, mit den Merkmalen des Anspruchs 1.

Die Erfindung löst die Aufgaben ebenfalls durch eine Vorrichtung zur Detektion von Fremdstoffenin Wasser, mit den Merkmalen des Anspruchs 9.

Einer der damit erzielten Vorteile ist, dass insbesondere mittels der Probeaufbereitung und der spektroskopischen Analyse der aufbereiteten Probe gemäß Schritt c) eine äußerst zuverlässige Ermittlung der Fremdstoffe ermöglicht wird: Durch das Aufbereiten der Probe mittels Destillation können Fremdstoffe selektiv getrennt und analysiert werden. Störstoffe, beispielsweise Huminstoffe, welche eine direkte Detektion von Fremdstoffen erschweren oder sogar verhindern, werden so ebenfalls quantitativ getrennt. Das Aufbereiten der Probe ermöglicht somit im Wesentlichen eine Abtrennung der eine Detektion der Fremdstoffe störenden Wassermatrix. Gleichzeitig kann durch die spektroskopische Analyse, insbesondere wenn die spektroskopische Analyse anhand von Absorptionsspektren erfolgt, auch aufgrund der charakteristischen Linien der jeweiligen Fremdstoffe in den aufgenommenen Spektren eine Vielzahl möglicher Fremdstoffe im Wasser zuverlässig unterschieden werden. Ebenso ermöglicht das Verfahren bzw. die Vorrichtung gemäß dem Anspruch 1 bzw. 9 eine im Wesentlichen kontinuierliche Überwachung der Fremdstoffe in Wasser, da sowohl der zeitliche Aufwand als auch der Material- und personelle Aufwand deutlich gesenkt wird. So wird mittels der vorliegenden Erfindung die Zeit von der Probenahme bis zum Erhalt des Analyseergebnisses auf weit unter eine Stunde, insbesondere auf weniger als 15 Minuten gesenkt. Ein Laser oder eine Deuteriumlampe als Lichtquelle ist dabei auf Grund einer hohen Emission im relevanten Spektralbereich besonders vorteilhaft.

Weitere vorteilhafte Ausführungsformen, Merkmale und Vorteile der Erfindung sind in den Unteransprüchen beschrieben.

Zweckmäßigerweise erfolgt zumindest das Analysieren mittels UV/VIS-Spektroskopie und/oder Fluoreszenz-Spektroskopie und es wird ein Absorptionsspektrum und/oder ein Fluoreszenz-Spektrum gemäß Schritt c2) aufgenommen. Die UV/VIS-Spektroskopie beruht auf der spezifischen Absorption von elektromagnetischer Strahlung in den Wellenlängenbereichen zwischen 1 nm und 800 nm, insbesondere zwischen 150 nm und 800 nm. Eine Analyse mittels UV/VIS-Spektroskopie hat den Vorteil, dass insbesondere organische Verbindungen zuverlässig detektiert bzw. ermittelt werden können. Darüber hinaus ist eine UV/VIS-Absorptionsmessung besonders gut automatisierbar, robust und erlaubt eine Ermittlung bzw. Detektion einer großen Anzahl organischer Verbindungen im Wasser. Ebenso wird damit eine ausreichende Genauigkeit bei der Detektion von Fremdstoffen in Wasser erreicht. Erfolgt das Analysieren mittels Fluoreszenz-Spektroskopie, hat dies den Vorteil, dass Fluoreszenz-Spektroskopie eine sehr spezifische Detektion von Fremdstoffen ermöglicht bei ausreichender Genauigkeit.

Vorteilhafterweise umfasst das Ermitteln gemäß Schritt d) die Verwendung von multivariaten Kalibrationsmethoden, chemometrischen Methoden und/oder einen Vergleich der aufgenommenen Spektren mit Referenzspektren. Unter multivariater möglicht eine zeitnahe und zuverlässige Ermittlung der Fremdstoffe im Wasser anhand des Vergleichs zwischen Referenzspektren und aufgenommenen Spektren.

Zweckmäßigerweise wird die multivariate Kalibration mittels der Teilweisen-Kleinste-Quadrate-Regression durchgeführt. Die als auch partial least squares (PLS-Regression) bekannte Regression ermöglicht ein besonders zuverlässiges Ermitteln von unterschiedlichen Fremdstoffen anhand des oder der aufgenommenen Spektren. Wird eine PLS-Regression durchgeführt werden allgemein Informationen eines Absorptionsspektrums eines Fremdstoffes im Wasser mit entsprechenden Konzentrationen verglichen. Hierzu wird eine bestimmte Anzahl von Spektren gemessen, um Veränderungen in den Spektren und bei den Konzentrationen zu erkennen und miteinander zu verknüpfen. Mathematisch werden Datenpunkte des Spektrums und der Konzentrationen in Matrizen dargestellt und dann in Eigenvektoren, sogenannte Faktoren zerlegt. Diese Faktoren werden nach sinkendem Eigenwert sortiert. Kleinere Eigenwerte kennzeichnen oder beschreiben im Allgemeinen Veränderungen des Systems Spektrum-Konzentration, wohingegen größere Eigenwerte im Allgemeinen spektrales Rauschen repräsentieren. Die Anzahl der Faktoren darf nicht zu gering sein, um noch genügend spektrale Eigenschaften des Systems abbilden zu können. Bei der PLS-Regression werden nun die ursprünglichen Spektral- und Konzentrationsmatrizen als Summe von Produkten aus sogenannten Scores- und Loading-Vektoren dargestellt. Im Falle von Absorptionsspektren werden identische Scores-Vektoren für die jeweiligen Faktorenzahlen in den Datensätzen für das Spektrum und für die Konzentration angenommen. Diese werden so gewählt, dass diese möglichst wenig von ihren jeweiligen ursprünglich berechneten Werten abweichen. Anschließend kann ein Regressionskoeffizient B berechnet werden, der im Wesentlichen den Zusammenhang zwischen Konzentration und Spektrum wiedergibt. Wird dann ein weiteres Spektrum aufgenommen, kann mittels des Spektrums und dem Regressionskoeffizienten die Konzentration des jeweiligen Fremdstoffes im Wasser berechnet werden. Anhand eines RMSECV-Wertes ("root mean square error of cross validation"- Wert) kann die Vorhersagegenauigkeit des erstellten Modells mittels der PLS-Regression ermittelt werden.

Insgesamt besteht der Schritt d) insbesondere aus sechs Unterschritten: Zunächst werden jeweilige Spektral- und Konzentrationsdaten ermittelt. In einem zweiten Schritt können die jeweiligen Daten vorbehandelt werden, um beispielsweise Drifts oder dergleichen zu eliminieren. In einem dritten Schritt werden geeignete Frequenz- oder Wellenlängenbereiche des Spektrums ausgewählt, die eine gute Korrelation zwischen Spektrum und Konzentration aufweisen. In einem vierten Schritt wird das so erhaltene Modell validiert und optimiert und ggf. "Ausreißer" entfernt. Danach werden in einem fünften Schritt die Kalibrationsfunktion oder Regressionskoeffizient B berechnet. In einem sechsten Schritt können dann neue Spektren mit Hilfe des erhaltenen Modells analysiert werden.

Vorteilhafterweise wird die entnommene Probe angesäuert, insbesondere mittels Zitronensäure und/oder Schwefelsäure, alkalisiert, insbesondere mittels Natriumhydroxid und/oder Soda, und/oder neutralisiert. Für eine zuverlässige Probenaufbereitung, insbesondere Destillation, ist vorzugsweise wenn die Fremdstoffe in Form von organischen Verbindungen oder Molekülen vorliegen, eine möglichst hohe Flüchtigkeit von Vorteil. Falls diese organischen Moleküle in ionischer Form vorliegen, und sich in eine ungeladene Molekülform überführen lassen, steigt deren Flüchtigkeit im Wasser an, was die Destillation und damit die Aufbereitung der entnommenen Wasserprobe wesentlich verbessert.

Zweckmäßigerweise werden der entnommenen Probe Komplexbilder hinzugefügt und/oder Mittel zur Derivatisierung. Einer der Vorteile beim Zusatz von Komplexbildern ist, dass damit eine Verkalkung der Probe im Neutralen verhindert werden kann. Als Komplexbildner kann beispielsweise Ethylendiamintetra-essigsäure oder dergleichen verwendet werden. Werden Mittel zur Derivatisierung der entnommenen Probe zugesetzt, kann eine Derivatisierung, insbesondere in einem Verdampfer erfolgen und es kann damit die Flüchtigkeit und/oder eine Verbesserung der Absorption ermöglicht werden, sodass insgesamt die Zuverlässigkeit der Detektion von Fremdstoffen im Wasser noch weiter verbessert wird.

Zweckmäßigerweise wird die Destillation gemäß Schritt b) in Form einer Vakuum-Destillation oder einer Überdruck-Destillation durchgeführt. Die Vakuumdestillation bietet neben einer Destillation bei Normaldruck den Vorteil, dass bei Wasser, insbesondere in Form von Trinkwasser die Kalkbildung, beispielsweise in einem Verdampfer, reduziert werden kann, wohingegen bei der Überdruck-Destillation eine erhöhte Anreicherung von Fremdstoffen in Form von Pflanzenschutzmitteln im Vergleich zur Destillation bei Umgebungsdruck oder bei der Vakuum-Destillation ermöglicht wird.

Vorteilhafterweise wird das Spektrum gemäß Schritt c2) in einem Wellenlängenbereich zwischen 50 nm und 1500 nm, vorzugsweise zwischen 100 nm und 1200 nm, insbesondere zwischen 200 nm und 1050 nm, besonders vorzugsweise zwischen 275 nm und 400 nm aufgenommen. Der damit erzielte Vorteil ist, dass in dem Wellenlängenbereich insbesondere Moleküle mit Doppelbindungen und aromatische Moleküle absorbieren und somit anhand von Absorptionsspektren zuverlässig detektierbar bzw. ermittelbar sind. Auf diese Weise können zuverlässig insbesondere Pestizide oder Pflanzenschutzmittel detektiert werden. Darüber hinaus können auch konjugierte Doppelbindungen sowie Carbonyle und andere funktionelle Gruppen analysiert und die entsprechenden Gruppen detektiert werden, was die Ermittlung des zumindest einen Fremdstoffs noch weiter verbessert.

Vorteilhafterweise erfolgt eine Anregung gemäß Schritt c1) in einem Wellenlängenbereich zwischen 50 nm und 600 nm, insbesondere zwischen 100 nm und 500 nm, vorzugsweise zwischen 150 nm und 400 nm. Damit ist es möglich, eine Anregung insbesondere für Fremdstoffe zu ermöglichen, welche im Trinkwasser, wie beispielsweise Pflanzenschutzmittel, etc. regelmäßig vorkommen, so dass ein aussagekräftiges Spektrum insbesondere für die Absorptionsspektroskopie zur Analyse und Erkennung der Fremdstoffe in der Probe erhalten werden kann. Gleiches gilt auch für die Fluoreszenz- und/oder die IR-Spektroskopie, insbesondere für die NIR-Spektroskopie. Die NIR-Spektroskopie erstreckt sich insbesondere im Bereich zwischen 700 nm und 3000 nm, vorzugsweise zwischen 800 nm und 2500 nm, insbesondere im Bereich zwischen 800 nm und 1500 nm, besonders vorzugsweise im Bereich von 1000 nm.

Vorteilhafterweise ist bei einer Vorrichtung gemäß Anspruch 10 eine Entnahmeeinrichtung zum Entnehmen einer Probe aus dem zu untersuchenden Wasser angeordnet. Der damit erzielte Vorteil ist, dass damit insbesondere automatisiert und periodisch, beispielsweise im Falle des Trinkwassers aus einer Leitung, eine Probe im Wesentlichen kontinuierlich der Vorrichtung zugeführt werden kann und die so entnommene Probe regelmäßig und automatisiert auf Fremdstoffe untersucht werden kann.

Zweckmäßigerweise ist eine Hilfsmittelzusatzeinrichtung zum Säuern, Alkalisieren, Neutralisieren der Probe und/oder zum Hinzufügen von Komplexbildnern und/oder zur Derivatisierung angeordnet. Für eine zuverlässige Probenaufbereitung, insbesondere Destillation, ist insbesondere, wenn die Fremdstoffe in Form von organischen Verbindungen oder Molekülen vorliegen, eine möglichst hohe Flüchtigkeit von Vorteil. Falls diese organischen Moleküle in ionischer Form vorliegen, und sich in eine ungeladene Molekülform überführen lassen, steigt deren Flüchtigkeit im Wasser an, was die Destillation und damit die Aufbereitung der entnommenen Probe wesentlich verbessert. Einer der Vorteile beim Zusatz von Komplexbildern ist, dass damit eine Verkalkung der Probe im Neutralen verhindert werden kann. Als Komplexbildner kann beispielsweise Ethylendiamintetra-essigsäure oder dergleichen verwendet werden. Werden Mittel zur Derivatisierung der entnommenen Probe zugesetzt, kann eine Derivatisierung, insbesondere in einem Verdampfer erfolgen und es kann damit die Flüchtigkeit und/oder eine Verbesserung der Absorption ermöglicht werden, sodass insgesamt die Zuverlässigkeit der Detektion von Fremdstoffen im Wasser verbessert wird.

Zweckmäßigerweise umfasst die Analysevorrichtung eine optische Meßzelle, welche in Form eines optischen Hohlleiters ausgebildet ist. Ein optischer Hohlleiter respektive eine Hohlleiter-Küvette ist eine flüssigkeitsführende Kapillare, die insbesondere von Innen mit einem reflektierenden Material beschichtet ist. Durch die Innenbeschichtung, welche einen höheren Berechnungsindex als die zu untersuchende Probe aufweist, wird der radiale Lichtaustritt gering gehalten. Durch Totalreflektion an der Grenzschicht zwischen Probe und Innenbeschichtung, wird somit der Lichtaustritt aus dem Probenlichtwellenleiter insgesamt verringert. Die Reflexionen haben zudem den Vorteil, dass die optische Weglänge vergrößert wird und somit die Nachweisempfindlichkeit erhöht wird. Ein weiterer Vorteil eines optischen Hohlleiters ist, dass dieser ein sehr geringes Probenvolumen erfordert, was die Aufbereitung der entsprechenden Probe verkürzt und weiter vereinfacht.

Zweckmäßigerweise ist die optische Meßzelle aus Quarz und/oder Edelstahl hergestellt. Einer der damit erzielten Vorteile ist, dass damit eine zuverlässige und gleichzeitig kostengünstige Analysevorrichtung mit optischer Meßzelle zur Verfügung gestellt werden kann. Ein weiterer Vorteil ist, dass eine Detektion von Fremdstoffen mittels Floureszenz-Spektroskopie äußerst zuverlässig möglich ist.

Vorteilhafterweise ist eine Steuereinrichtung zum Steuern der Vorrichtung angeordnet. Die Steuervorrichtung ermöglicht eine automatisierte und prozessgesteuerte Durchführung insbesondere des Verfahrens gemäß einem der Ansprüche 1 bis 8 mittels der Vorrichtung gemäß einem der Ansprüche 9 bis 14.

Weitere wichtige Merkmale und Vorteile der Erfindung ergeben sich aus den Unteransprüchen, aus den Zeichnungen, und aus der dazugehörigen Figurenbeschreibung anhand der Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungen und Ausführungsformen der Erfindung sind in den Zeichnungen dargestellt und werden in der nachfolgenden Beschreibung näher erläutert, wobei sich gleiche Bezugszeichen auf gleiche oder ähnliche oder funktional gleiche Bauteile oder Elemente beziehen.

Dabei zeigen jeweils in schematischer Form
- Fig. 1: eine Vorrichtung gemäß einer ersten Ausführungsform der vorliegenden Erfindung;
- Fig. 2: Schritte eines Verfahrens gemäß einer zweiten Ausführungsform der vorliegenden Erfindung;
- Fig. 3: Absorptionsspektren von Nitrobenzol aus Trinkwasser;
- Fig. 4a, 4b, 4c: eine Wertetabelle und Schaubilder einer Eigenabsorption von destilliertem Wasser im UV-Bereich in verschiedenen Maßstäben,
- Fig. 5: Fluoreszenzspektren von Bentazon in verschiedenen Konzentrationen aus demineralisiertem Wasser;
- Fig. 6: Absorptionsspektren verschiedener Pestizide;
- Fig. 7: Absorptionsspektren von Atrazin bei verschiedenen Überdrücken;
- Fig. 8a: Wiederfindungsraten von Nitrobenzol in Trinkwasser; und
- Fig. 8b: statistische Werte für die Wiederfindungsraten gemäß Fig. 8a.

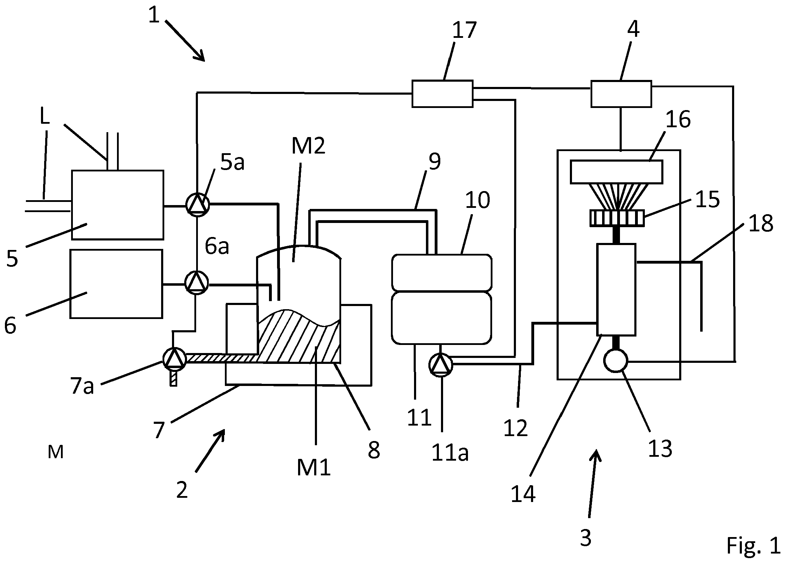

Fig. 1 zeigt eine Vorrichtung gemäß einer ersten Ausführungsform der vorliegenden Erfindung.

In Fig. 1 bezeichnet Bezugszeichen 1 eine Vorrichtung zur Detektion von Fremdstoffen in Trinkwasser. Die Vorrichtung 1 umfasst dabei eine Aufbereitungseinrichtung 2 und eine Analyseeinrichtung 3. Die Aufbereitungseinrichtung 2 umfasst einen Behälter 8, der mittels einer Heizung 7 erwärmt werden kann. Weiterhin ist eine Entnahmeeinrichtung 5 angeordnet, welche mittels einer Pumpe 5a eine Probe von Trinkwasser M aus einer Trinkwasserleitung L entnehmen und mittels einer Pumpe 5a in den Behälter 8 fördern kann. Um die im Behälter 8 befindliche Probe des Trinkwassers M dann aufbereiten, insbesondere zusätzlich neutralisieren, zu können, ist ein Hilfsmittelreservoir 6 vorgesehen, welches Hilfsmittel über eine Pumpe 6a in den Behälter 8 fördern kann, so dass das Hilfsmittel mit der entnommenen Probe vermischt werden kann. Hierfür können entsprechende Vermischungseinrichtungen, wie Rührer, etc. ebenfalls angeordnet sein. Die entnommene Probe des Trinkwassers M wird dann mittels einer Heizung 7 erhitzt, so dass ein Teil des Trinkwassers M in die Gasphase überführt wird (Bezugszeichen M2). Ein Rest M1 des Trinkwassers M verbleibt in der flüssigen Phase.

Über eine Pumpe 7a, die über Leitungen mit dem Behälter 8 verbunden ist, kann der nicht verdampfte und nicht mehr benötigte flüssige Rest M1 des Trinkwassers M später entsorgt werden. Das verdampfte Trinkwasser M2 wird über eine Leitung 9 einem Kühler 10 zugeführt, in dem dieses wiederum kondensiert. Anschließend wird es in einem Sammler 11 gesammelt und mittels einer Pumpe 11a über eine Leitung 12 in eine Küvette 14 transportiert. Die Küvette 14 kann dabei insbesondere als optische Hohlleiter-Küvette ausgebildet ist sein und wird dann längs ihrer Achse mit Licht einer Lichtquelle 13 durchstrahlt. Auf der der Lichtquelle 13 gegenüberliegenden Seite der Küvette 14 wird das aus der Küvette 14 austretende Licht wellenlängenspezifisch, beispielsweise mittels eines Prismas 15 aufgespalten. Das so aufgespaltene Licht wird auf einen Detektor 16 geleitet, der mit einer Ermittlungseinrichtung 4 verbunden ist. Die Ermittlungseinrichtung 4 vergleicht dann das mittels des Detektors 16 aufgenommene Spektrum der Probe in der Küvette 14 mit in der Ermittlungseinrichtung 4 hinterlegten Referenzspektren für die jeweiligen zu analysierenden Fremdstoffe und kann diese die ermittelten Fremdstoffe dann gegebenenfalls einem Benutzer der Vorrichtung signalisieren oder anzeigen. Mittels eines Ablasses 18 kann dann der Inhalt der Küvette 14, wenn dieser zur Messung nicht mehr benötigt wird, entsorgt werden. Selbstverständlich kann die Ermittlungseinrichtung 4 auch ein entsprechendes Modell zur Ermittlung der Fremdstoffe in Wasser mittels multivariater Methoden und mittels neuronaler Netze erstellen, um die Fremdstoffe zu detektieren.

Gleichzeitig kann die Ermittlungseinrichtung 4 ebenfalls mit einer Steuerungseinrichtung 17 verbunden sein. Die Steuerungseinrichtung 17 dient zur Steuerung der Vorrichtung 1 und ist insbesondere wie in Fig. 1 gezeigt mit den jeweiligen Pumpen 5a, 6a, 7a und 11a verbunden, ebenso wie mit der Ermittlungseinrichtung 4. Die Steuerungseinrichtung 17 ermöglicht dann einen automatisieren Ablauf, indem beispielsweise zunächst die Pumpe 5a betätigt wird und dann gegebenenfalls entsprechend Hilfsmittel über die Hilfsmittelpumpe 6a zugeführt wird. Anschließend wird mittels der Steuerungseinrichtung 17 die Heizung 7 betätigt und ein Teil der entnommenen Probe des Trinkwassers M in dem Behälter 8 entsprechend verdampft. Die Steuerungseinrichtung 17 kann ebenfalls über entsprechende Sensoren im Sammler 11 ermitteln, ob bzw. inwieweit genügend Kondensat bzw. Destillat erzeugt wurde. Ist genügend Destillat im Sammler 11 vorhanden, wird mittels der Steuerungseinrichtung 17 die Heizung 7 abgeschaltet und mittels der Pumpe 11a und der Leitung 12 das Destillat in die Küvette 14 gefördert. Die Steuerungseinrichtung 17 steuert dann die Ermittlungseinrichtung 4, welche wiederum die Lichtquelle 13 und den Detektor 16 betätigt. Hat der Detektor 16 ein entsprechendes Spektrum des in der Küvette 14 befindlichen destillierten Anteils der entnommenen Probe aufgenommen, übermittelt er dieses an die Ermittlungseinrichtung 4, die wiederum die Detektion bzw. Ermittlung der Fremdstoffe vornimmt. Ist das Ergebnis nicht eindeutig oder mit zu vielen Fehlern behaftet, kann die Ermittlungseinrichtung 4 ein entsprechendes Signal an die Steuerungseinrichtung 17 übermitteln, welche dann erneut eine Probe aus der Trinkwasserleitung L entnehmen kann. Vorher wird jedoch der nicht verdampfte Teil der Probe des Trinkwassers M in dem Behälter 8 mittels Betätigung der Pumpe 7a über die Steuerungseinrichtung 17 entleert. Für eine neue Ermittlung kann die Steuerungseinrichtung 17 ebenfalls das Leitungssystem der Vorrichtung 1 ganz oder teilweise mit einer vordefinierten Flüssigkeit, beispielsweise destilliertem Wasser spülen, um Reste verbliebener Fremdstoffe zu entfernen, um eine zuverlässige Messung von weiteren Proben zu gewährleisten.

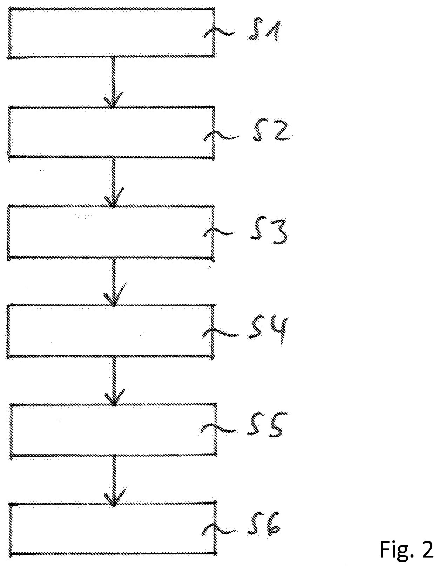

Fig. 2 zeigt Schritte eines Verfahrens gemäß einer zweiten Ausführungsform der vorliegenden Erfindung. In Fig. 2 bezeichnet Bezugszeichen S1 den Schritt: Entnehmen einer Probe, Bezugszeichen S2 den Schritt: Aufbereiten der entnommenen Probe, wobei das Aufbereiten der Probe eine zumindest teilweise Destillation der entnommenen Probe umfasst, Bezugszeichen S3 den Schritt: Analysieren der aufbereiteten Probe mittels Absorptions- und/oder Emissions-Spektroskopie, Bezugszeichen S4 den Schritt: Anregen der aufbereiteten Probe mit Licht, insbesondere eines Lasers oder einer Deuteriumlampe, mit zumindest einer Anregungswellenlänge, Bezugszeichen S5 den Schritt: Aufnehmen zumindest eines Absorptions- und/oder Emissionsspektrums des durch die Probe transmittierten und/oder des von der Probe emittierten Lichts mittels eines Detektors, und Bezugszeichen S6 den Schritt: Ermitteln zumindest eines der Fremdstoffe in der analysierten Probe anhand zumindest eines der aufgenommenen Absorptions- und/oder Emissionsspektren.

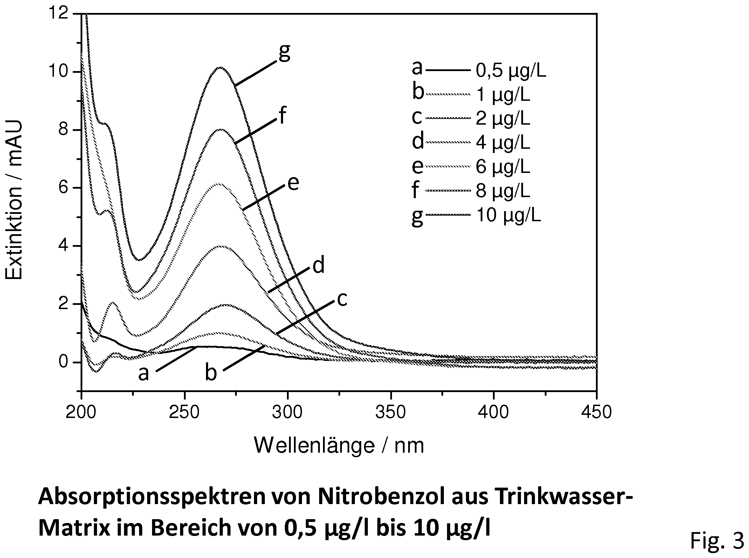

Fig. 3 zeigt Absorptionsspektren von Nitrobenzol aus Trinkwasser.

In Fig. 3 sind Absorptionsspektren von verschiedenen Konzentrationen von Nitrobenzol in einem Trinkwasser gezeigt. Die Wellenlänge der aufgenommenen Absorptionsspektren erstreckt sich dabei zwischen 200 nm und 450 nm auf der horizontalen Achse. Auf der vertikalen Achse ist die Extinktion in Einheiten von Milliabsorptionseinheiten (mAU) im Wesentlichen zwischen 0 und 12 angegeben. Der Kurvenverlauf insbesondere bei Konzentrationen von Nitrobenzol oberhalb von vier Mikrogramm pro Liter im Trinkwasser (Kurven mit Bezugszeichen d, e, f, g) ist dabei im Wesentlichen wie folgt: Lokales Minimum bei ca. 1,5, 2, 2,25 und 3,5 mAU bei ca. 227 nm Wellenlänge, anschließender Anstieg auf ein lokales Maximum bei ca. 4, 6, 8, bzw. 10 mAU bei ca. 265 nm Wellenlänge und anschließend rasches Abklingen auf ca. 1 mAU bei ca. 325 nm und schließlich weiteres Abklingen auf ca. 0 mAU oberhalb von 400 nm Wellenlänge. Auch bei kleineren Konzentrationen zwischen 0,5 Mikrogramm pro Liter (Bezugszeichen a) bis 2 Mikrogramm pro Liter (Kurve mit Bezugszeichen c) ist ein lokales Maximum bei ca. 265 nm zu sehen, dieses ist jedoch bei der Kurve a) kaum ausgeprägt.

Diese Absorptionsspektren können dann als Referenzspektren für Nitrobenzol dienen und bei anderen Konzentrationen entsprechend interpoliert werden.

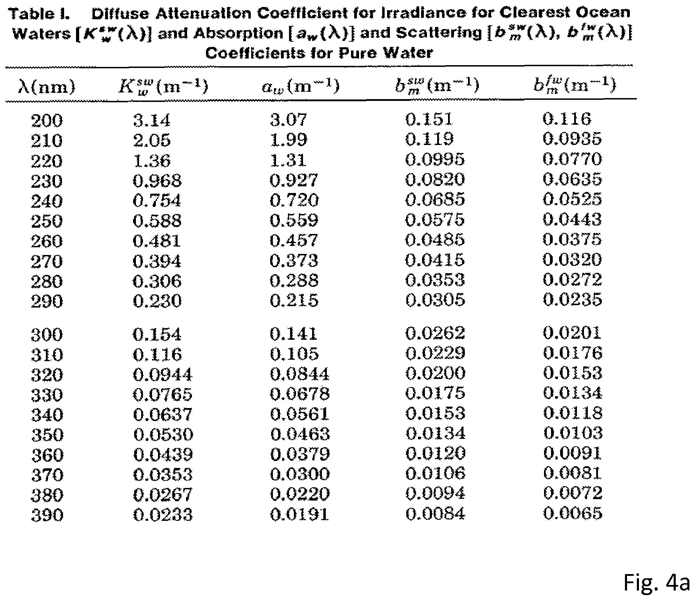

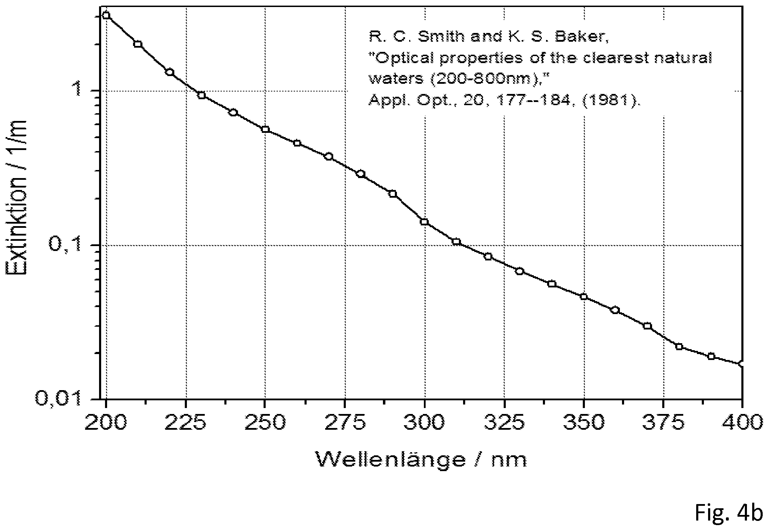

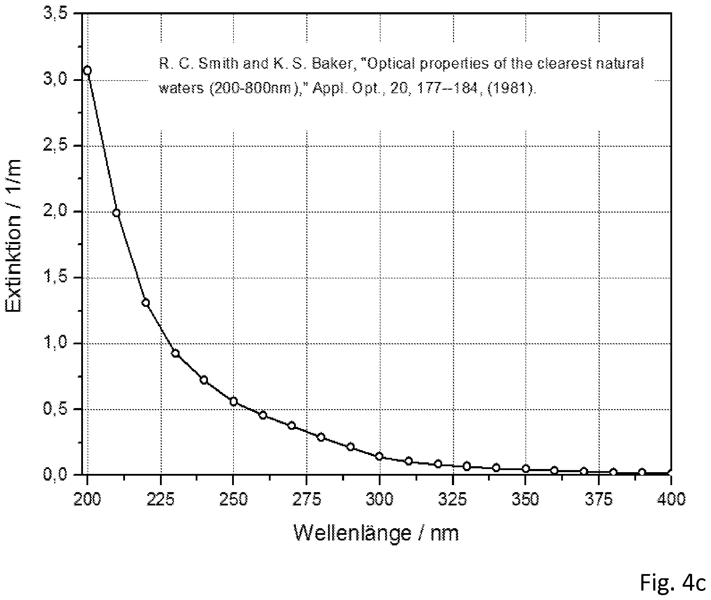

Fig. 4a, 4b und 4c zeigen eine Wertetabelle sowie Schaubilder einer Eigenabsorption von destilliertem Wasser im UV-Bereich in verschiedenem Maßstab.

Fig. 4a zeigt eine Wertetabelle des Eigenabsorptionsspektrums von destilliertem Wasser, Fig. 4b zeigt das zugehörige Schaubild mit halblogarithmischer Auftragung auf der vertikalen Achse im Bereich zwischen 200 nm und 400 nm und Fig. 4c das zugehörige Schaubild entsprechend in linearer Auftragung. Die Schaubilder wurden aus R.C. Smith and K.S. Baker, "Optical Properties of the clearest natural waters (200-800nm)", Appl. Opt. 20, 177-184(1981) entnommen.

Zu erkennen ist insbesondere in den Fig. 4b und Fig. 4c, dass die Absorption von destilliertem Wasser insbesondere im Wellenlängenbereich zwischen 200 nm und 225 nm relativ hoch ist, d. h., dass beispielsweise bei 1m Schichtdicke in diesem Bereich nur noch mit einem Zehntel bzw. einem Hundertstel des mittels einer Lichtquelle auf eine Probe destillierten Wassers eingestrahlten Lichts auf den Detektor treffen kann. Zur Fluoreszenz-Anregung der in der Küvette befindlichen Probe muss die Lichtquelle dementsprechend in dem Bereich zwischen 200 nm und 225 nm eine starke Emission aufweisen, damit noch genügend Licht auf den Detektor trifft. Hierfür eignen sich insbesondere ein Laser oder eine Deuteriumlampe. Daneben ist eine empfindliche und rauscharme Detektion vorteilhaft. Beides gilt auch für Absorptionsmessungen auf Grund der erhöhten Eigenabsorption von Wasser im relevanten Wellenlängenbereich.

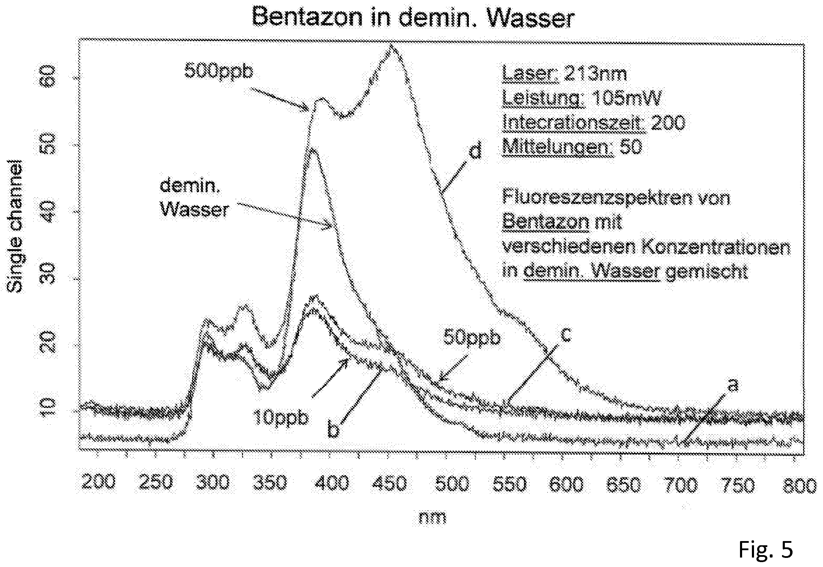

Fig. 5 zeigt Fluoreszenzspektren von Bentazon in verschiedenen Konzentrationen in demineralisiertem Wasser.

In Fig. 5 ist ein Fluoreszenzspektrum zwischen 200 nm und 850 nm von Bentazon in demineralisiertem Wasser gezeigt. Das Bentazon wurde mittels eines Lasers mit einer Wellenlänge von 213 nm und einer Leistung von 105 mW angeregt. Bezugszeichen a in Fig. 5 bezeichnet den Verlauf des Fluoreszenzspektrums von demineralisiertem Wasser. Die Kurve a weist dabei im Wesentlichen ein lokales Maximum im Bereich von 385 nm auf. Dieses lokale Maximum ist deutlich höher als die lokalen Maxima der Kurven b und c für Konzentrationen von Bentazon in demineralisiertem Wasser von 10 µg/L (Kurve b) und 50 µg/L (Kurve c). Darüber hinaus weisen die Kurven b und c einen deutlich schwächeren Abfall im Bereich zwischen 450 nm und 500 nm auf. Hier fällt die Kurve a deutlich stärker und unterschreitet die beiden Kurven b und c. Die Kurve d repräsentiert das Fluoreszenzspektrum von Bentazon in demineralisiertem Wasser bei einer Konzentration von Bentazon von 500 µg/L. Diese Kurve d unterscheidet sich erheblich von der Kurve von demineralisiertem Wasser (Kurve a) und weist beispielsweise bei 450 nm einen absoluten Höhepunkt auf. Aufgrund der charakteristischen Merkmale von Bentazon in demineralisiertem Wasser im Wellenlängenbereich zwischen 350 nm und 500 nm ist eine zuverlässige Detektion von Bentazon und eine Unterscheidung verschiedener Fremdstoffe in einem flüssigen Medium aufgrund des Fluoreszenzspektrums möglich.

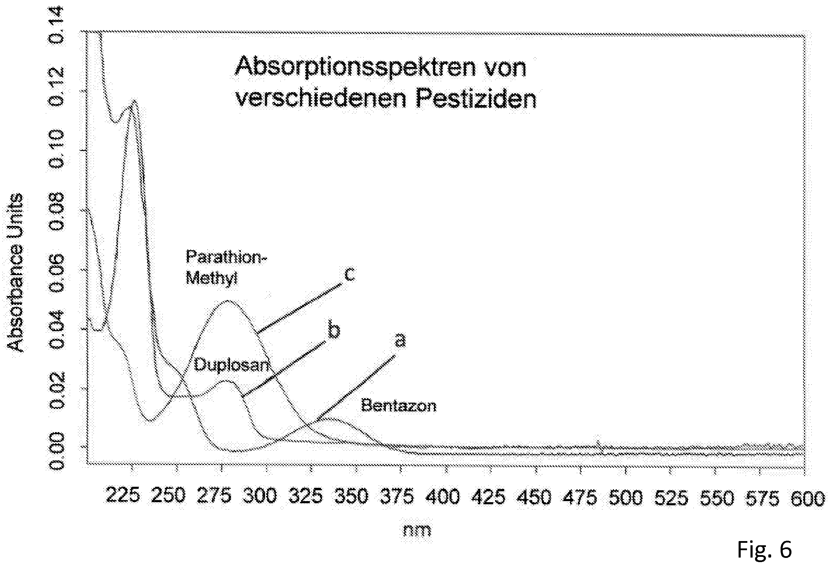

Fig. 6 zeigt Absorptionsspektren verschiedener Pestizide.

In Fig. 6 sind Absorptionsspektren zwischen 200 nm und 600 nm der Pestizide Bentazon, Duplosan und Parathion-Methyl gezeigt. Duplosan (Kurve b) und Bentazon (Kurve a) weisen bei ca. 230 nm einen charakteristischen ersten Peak oder lokales Maximum von ca. 0,11 AU auf. Bentazon weist im weiteren Verlauf noch ein lokales Maximum von 0,01 AU bei ca. 337 nm auf, wohingegen Duplosan ein weiteres lokales Maximum bei ca. 280 nm aufweist. Parathion-Methyl (Kurve c) weist ebenfalls bei ca. 280 nm ein lokales Maximum von 0,045 AU auf, weist jedoch im Bereich von 235 nm ein lokales Minimum von 0,15 AU auf. Auf diese Weise lassen sich dann verschiedene Pestizide anhand ihrer charakteristischen Absorptionsspektren zuverlässig unterscheiden, beispielsweise indem entsprechende Referenzspektren hinterlegt und die gemessenen Spektren mit diesen verglichen werden.

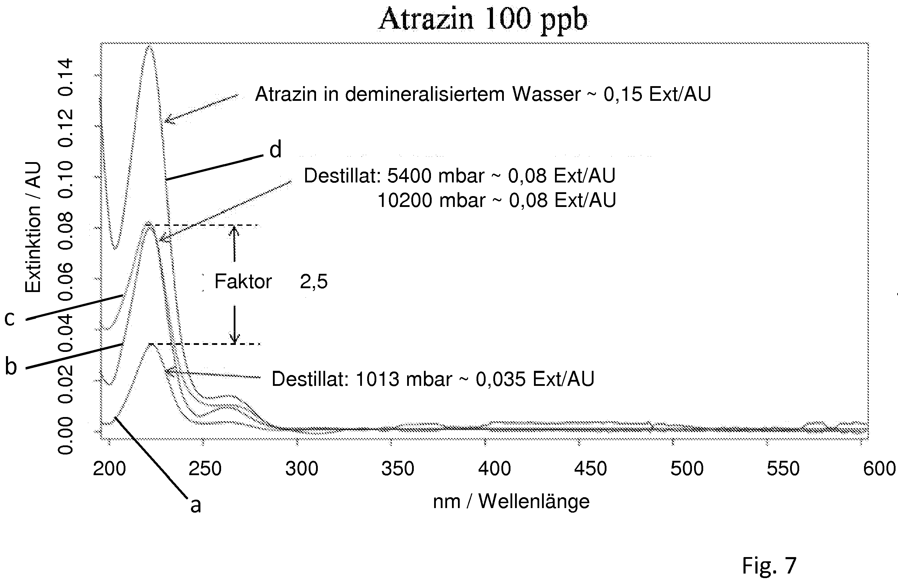

Fig. 7 zeigt Absorptionsspektren von Atrazin bei verschiedenen Überdrücken.

In Fig. 7 sind Absorptionsspektren von Atrazin nach Überdruckdestillation gezeigt. Ausgangspunkt hierfür war demineralisiertes Wasser, welchem Atrazin in einer Konzentration von 100 µg/L zugesetzt wurde (Absorptionsspektrum Kurve d). Dieses wurde dann bei einem Umgebungsdruck von 1013 mbar destilliert und das Destillat einer Absorptionsspektralanalyse unterzogen. Es ergab sich wie in Fig. 7 gezeigt ein charakteristischer Peak bei ca. 225 nm und einer Höhe von 0,035 Absorptionseinheiten (Kurve a). Anschließend wurde die Ausgangsprobe von Atrazin in einer Konzentration von 100 µg/L in demineralisierten Wasser unter Überdruck, einmal bei 5400 mbar (Kurve b) und bei 10200 mbar (Kurve c) wiederholt. Beide Kurven b und c weisen wie auch die Kurve a einen charakteristischen Peak bei ca. 225 nm auf, jedoch ist der Peak der Kurven b) und c) nun 0,08 Absorptionseinheiten hoch und damit um den Faktor 2,5 erhöht. Mittels der Überdruckdestillation konnte somit eine Aufkonzentrierung des Fremdstoffes in Form von Atrazin vorgenommen werden.

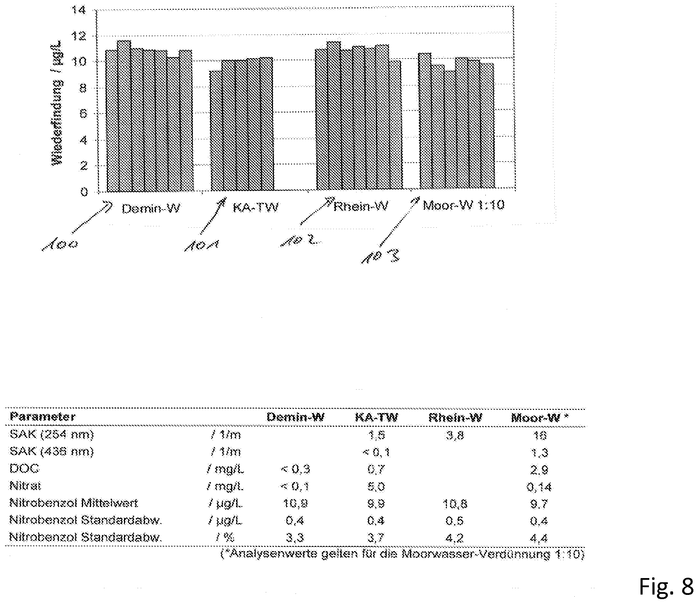

Fig. 8a zeigt Wiederfindungsraten von Nitrobenzol in Trinkwasser.

In Fig. 8a bezeichnet Bezugszeichen 100 sieben verschiedene Wiederfindungsraten für Nitrobenzol bei einer Konzentration von 10 µg/L in demineralisiertem Wasser gemäß einer Ausführungsform des Verfahrens der vorliegenden Erfindung. Dabei liegen die Wiederfindungsfindungsraten zwischen 10 µg/L und 11,5 µg/L.

Bezugszeichen 101 bezeichnet fünf verschiedene Wiederfindungsraten für Nitrobenzol bei einer Konzentration von 10 µg/L in einer Karlsruher Trinkwasser-probe gemäß einer Ausführungsform des Verfahrens der vorliegenden Erfindung. Dabei liegen die Wiederfindungsfindungsraten zwischen 9,5 µg/L und 10,2 µg/L.

Bezugszeichen 102 bezeichnet sieben verschiedene Wiederfindungsraten für Nitrobenzol bei einer Konzentration von 10 µg/L in einer Rheinwasserprobe gemäß einer Ausführungsform des Verfahrens der vorliegenden Erfindung. Dabei liegen die Wiederfindungsfindungsraten zwischen 9,7 µg/L und 11,5 µg/L.

Bezugszeichen 103 bezeichnet sechs verschiedene Wiederfindungsraten für Nitrobenzol bei einer Konzentration von 10 µg/L in einer 1:10 verdünnten Moorwasserprobe gemäß einer Ausführungsform des Verfahrens der vorliegenden Erfindung. Dabei liegen die Wiederfindungsfindungsraten zwischen 9,5 µg/L und 10,5 µg/L.

Insgesamt ist damit sowohl die Qualität als auch die Stabilität des Verfahrens gemäß einer Ausführungsform der vorliegenden Erfindung ausreichend hoch und ermöglicht eine zuverlässige Detektion von Fremdstoffen in Wasser.

Fig. 8b zeigt statistische Werte für die Wiederfindungsraten gemäß Fig. 8a.

In Fig. 8b sind jeweils der spektrale Absorptionskoeffizient für 254 nm und 463 nm, der DOC-Wert, der Nitratwert, der jeweils ermittelte Mittelwert von Nitrobenzol, sowie die entsprechende absolute und relative Standardabweichung für die Wässer 100, 101, 102 und 103 gemäß Fig. 8a gezeigt. Insgesamt zeigen die ermittelten Werte für den jeweiligen Mittelwert von 10,9 µg/L, 9,9 µg/L, 10,8 µg/L, und 9,7 µg/L sowie geringe absolute und relative Standardabweichungen zwischen 0,4 und 0,.5 µg/L bzw. zwischen 3,3% und 4,4% die hohe Genauigkeit, Stabilität und Qualität des Verfahrens und/oder der Vorrichtung zur Detektion von Fremdstoffen in Wasser gemäß der vorliegenden Erfindung.

Zusammenfassend weist die vorliegende Erfindung mehrere Vorteile auf: Zum einen ist das Verfahren bzw. die Vorrichtung kostengünstig und mit einer hohen Genauigkeit für die Erkennung bzw. Detektion von Fremdstoffen in Wasser durchführbar bzw. herstellbar. Darüber hinaus kann mit der Erfindung eine Vielzahl möglicher Fremdstoffe in Wasser zuverlässig detektiert und ihre Konzentration bestimmt werden. Schließlich bietet die vorliegende Erfindung den Vorteil, dass das Verfahren automatisiert ablaufen kann und gleichzeitig der personelle und zeitliche Kostenaufwand wesentlich gesenkt wird. Die Zuverlässigkeit der Überwachung von Fremdstoffen in Wasser, insbesondere in Trinkwasser kann dadurch wesentlich erhöht werden.

Anstelle der Anreicherung der Fremdstoffe mittels Destillation ist ebenfalls eine Anreicherung der jeweiligen Fremdstoffe in Wasser mittels Festphasenextraktion, Flüssig-Flüssig-Extraktion, mittels Membranfiltration, insbesondere in Form einer Nanofiltration und/oder mittels Ionenaustauschern möglich. Diese Techniken können zusammen mit der Destillation alternativ oder zusätzlich zu dieser zur Verbesserung der Probenvor- oder -aufbereitung verwendet werden. Alternativ oder zusätzlich zur UV-/VIS-Spektroskopie und/oder Fluoreszenzspektroskopie kann ebenfalls Infrarot-Absorptionsspektroskopie zur Detektion im Bereich zwischen 0,6 µm und 600 µm, insbesondere zwischen 0,8 µm und 500 µm Wellenlänge verwendet werden, insbesondere FTIR-Absorptionsspektroskopie. Zur weiteren Verbesserung der Probenvorbereitung bzw. Aufbereitung kann die Probe zusätzlich entgast werden. Darüber hinaus kann die Analyseeinrichtung 3 ebenfalls eine Heizeinrichtung und entsprechende Sensoren umfassen, so dass alle Komponenten bzw. Bauteile der Analyseeinrichtung 3 auf derselben Temperatur gehalten werden, was die Ermittlung der Fremdstoffe im flüssigen Medium weiter verbessert. Darüber hinaus können regelmäßig sogenannte Nullmessungen durchgeführt werden, so dass die Vorrichtung automatisiert kalibriert werden kann und ein Driften in der Sensorik bzw. kleinste Temperaturschwankungen, etc. ausgeschlossen bzw. minimiert werden können.

Wird das Ermitteln der Fremdstoffe mittels aufgenommener Spektren und Referenzspektren vorgenommen, kann in der Ermittlungseinrichtung eine entsprechende Datenbank für die Spektren in Form von entsprechenden UV/-VIS-Spektren, Fluoreszenzspektren und/oder IR-Spektren mit jeweils verschiedenen Konzentrationen hinterlegt werden. Die Referenzspektren können dann beispielsweise in einer rein wässrigen Lösung gemessen werden und insbesondere mittels eines Zusatzes eines Puffers neutrale pH-Bedingungen für die Messung geschaffen werden.

Obwohl die vorliegende Erfindung vorstehend anhand bevorzugter Ausführungsbeispiele beschrieben wurde, ist sie nicht darauf beschränkt, sondern auf vielfältige Weise modifizierbar.

### Bezugszeichenliste

- 1: Vorrichtung zur Detektion von Fremdstoffen in Wasser
- 2: Aufbereitungseinrichtung
- 3: Analyseeinrichtung
- 4: Ermittlungseinrichtung
- 5: Entnahmeeinrichtung
- 5a: Pumpe
- 6: Hilfsmittelreservoir
- 6a: Pumpe
- 7: Heizung
- 7a: Pumpe
- 8: Behälter
- 9: Leitung
- 10: Kühler
- 11: Sammler
- 11a: Pumpe
- 12: Leitung
- 13: Lichtquelle
- 14: Küvette
- 15: Prisma
- 16: Detektor
- 17: Steuerungseinrichtung
- 18: Ablass
- M: Trinkwasser
- M1: Trinkwasser flüssige Phase
- M2: Trinkwasser Gasphase
- L: Trinkwasserleitung

## Patentansprüche

1. Verfahren zur Detektion von Fremdstoffen, insbesondere von Schadstoffen, in Wasser, insbesondere in Trinkwasser, vorzugsweise geeignet zur Durchführung mit einer Vorrichtung gemäß einem der Ansprüche 9-14, umfassend die Schritte
a) Entnehmen (S1) einer Probe (M),
b) Aufbereiten (S2) der entnommenen Probe (M), wobei das Aufbereiten der Probe eine zumindest teilweise Destillation (2) der entnommenen Probe (M) umfasst,
c) Analysieren des erhaltenen Kondensats mittels UV/VIS-Spektroskopie und/oder Fluoreszenz-Spektroskopie, wobei das Analysieren die folgenden Schritte umfasst:
c1) Anregen (S4) des Kondensats mit Licht (13), insbesondere eines Lasers oder einer Deuteriumlampe, mit zumindest einer Anregungswellenlänge, und
c2) Aufnehmen (S5) zumindest eines Absorptionsspektrums und/oder Fluoreszenzspektrums des durch das Kondensat transmittierten und/oder des von dem Kondensat emittierten Lichts mittels eines Detektors (16),
und
d) Ermitteln (S6) zumindest eines der Fremdstoffe in dem analysierten Kondensat (M) anhand zumindest eines der aufgenommenen Absorptions- und/oder Fluoreszenzspektren.

2. Verfahren gemäß Anspruch 1, **dadurch gekennzeichnet, dass** das Ermitteln (S6) gemäß Schritt d) die Verwendung von Multivariaten Kalibrationsmethoden, chemometrischen Methoden und/oder einen Vergleich der aufgenommen Spektren mit Referenzspektren umfasst.

3. Verfahren gemäß einem der Ansprüche 1-2, **dadurch gekennzeichnet, dass** die multivariate Kalibration mittels der Teilweisen-Kleinste-Quadrate-Regression durchgeführt wird.

4. Verfahren gemäß einem der Ansprüche 1-3, **dadurch gekennzeichnet, dass** die entnommene Probe angesäuert, insbesondere mittels Zitronensäure und/oder Schwefelsäure, alkalisiert, insbesondere mittels Natriumhydroxid und/oder Soda, und/oder neutralisiert wird.

5. Verfahren gemäß einem der Ansprüche 1-4, **dadurch gekennzeichnet, dass** der entnommenen Probe Komplexbildner und/oder Mittel zur Derivatisierung hinzugefügt werden.

6. Verfahren gemäß einem der Ansprüche 1-5, **dadurch gekennzeichnet, dass** die Destillation (2) gemäß Schritt b) in Form einer Vakuum-Destillation oder einer Überdruck-Destillation durchgeführt wird.

7. Verfahren gemäß einem der Ansprüche 1-6, **dadurch gekennzeichnet, dass** das Spektrum gemäß Schritt c2) in einem Wellenlängenbereich zwischen 50 nm und 1500 nm, vorzugsweise zwischen 100 nm und 1200 nm, insbesondere zwischen 200 nm und 1050 nm, besonders vorzugsweise zwischen 275 nm und 400 nm aufgenommen wird.

8. Verfahren gemäß einem der Ansprüche 1-7, **dadurch gekennzeichnet, dass** eine Anregung gemäß Schritt c1) in einem Wellenlängenbereich zwischen 50 nm und 600 nm, insbesondere zwischen 100 nm und 500 nm, vorzugsweise zwischen 150 nm und 400 nm erfolgt.

9. Vorrichtung ausgestaltet zur Detektion von Fremdstoffen, insbesondere von Schadstoffen, in Wasser, insbesondere in Trinkwasser, vorzugsweise zur Durchführung eines Verfahrens gemäß einem der Ansprüche 1-8, umfassend eine Aufbereitungseinrichtung (2) zur Aufbereitung (8) einer aus dem Wasser (M) entnommenen Probe, wobei die Aufbereitungseinrichtung (2) eine Destillationseinrichtung (7, 8, 9, 10, 11) zum zumindest teilweisen Destillieren der entnommenen Probe umfasst,
eine Analysiereinrichtung (3) zum Analysieren des erhaltenen Kondensats mittels UV/VIS-Spektroskopie und/oder Fluoreszenz-Spektroskopie, wobei die Analysiereinrichtung (3) zumindest eine Lichtquelle (13), insbesondere einen Laser, zum Anregen des Kondensats mit Licht mit zumindest einer Anregungswellenlänge umfasst und einen Detektor (16) zum Aufnehmen zumindest eines Absorptionsspektrums und/oder Fluoreszenzspektrums des durch das Kondensat transmittierten und/oder des von dem Kondensat emittierten Lichts, sowie
eine Ermittlungseinrichtung (4) zum Ermitteln zumindest eines der Fremdstoffe in dem Kondensat (M) anhand zumindest eines der aufgenommenen Absorptions- und/oder Fluoreszenzspektren.

10. Vorrichtung gemäß Anspruch 9, **dadurch gekennzeichnet, dass** eine Entnahmeeinrichtung (5) zum Entnehmen einer Probe aus dem zu untersuchenden Wasser (M) angeordnet ist.

11. Vorrichtung gemäß einem der Ansprüche 9-10, **dadurch gekennzeichnet, dass** eine Hilfsmittelzusetzeinrichtung (6) zum Säuern, Alkalisieren, Neutralisieren der Probe und/oder zum Hinzufügen von Komplexbildnern und/oder zur Derivatisierung angeordnet ist.

12. Vorrichtung gemäß einem der Ansprüche 9-11, **dadurch gekennzeichnet, dass** die Analyseeinrichtung (3) eine optische Meßzelle (14) umfasst, welche in Form eines optischen Hohlleiters ausgebildet ist.

13. Vorrichtung gemäß einem der Ansprüche 9-12, **dadurch gekennzeichnet, dass** die optische Meßzelle (14) aus Quarz und/oder Edelstahl hergestellt ist.

14. Vorrichtung gemäß einem der Ansprüche 9-13, **dadurch gekennzeichnet, dass** eine Steuereinrichtung (17) zum Steuern der Vorrichtung angeordnet ist.

## Claims

1. Method for detecting foreign substances, in particular harmful substances, in water, in particular in drinking water, preferably suitable for implementation using a device according to any one of claims 9 to 14, comprising the steps of:
a) removing (S1) a sample (M),
b) preparing (S2) the removed sample (M), wherein the preparation of the sample involves an at least partial distillation (2) of the removed sample (M),
c) analysing the condensate obtained by means of UV/VIS spectroscopy and/or fluorescence spectroscopy, wherein the analysis comprises the following steps:
c1) exciting (S4) the condensate with light (13), in particular of a laser or a deuterium lamp, having at least one excitation wavelength, and
c2) recording (S5) at least one absorption spectrum and/or fluorescence spectrum of the light transmitted by the condensate and/or the light emitted by the condensate by means of a detector (16),
and
d) establishing (S6) at least one of the foreign substances in the analysed condensate (M) using at least one of the recorded absorption and/or fluorescence spectrums.

2. Method according to claim 1, **characterised in that** the establishment (S6) comprises according to step d) the use of multivariate calibration methods, chemometric methods and/or a comparison of the recorded spectrums with reference spectrums.

3. Method according to either claim 1 or claim 2, **characterised in that** the multivariate calibration is carried out by means of the partial least squares regression.

4. Method according to any one of claims 1 to 3, **characterised in that** the sample removed is acidified, in particular by means of citric acid and/or sulphuric acid, is alkalised, in particular by means of sodium hydroxide and/or soda, and/or is neutralised.

5. Method according to any one of claims 1 to 4, **characterised in that** complexing agents and/or means for derivatisation are added to the removed sample.

6. Method according to any one of claims 1 to 5, **characterised in that** the distillation (2) is carried out according to step b) in the form of a vacuum distillation or an excess pressure distillation.

7. Method according to any one of claims 1 to 6, **characterised in that** the spectrum according to step c2) is recorded in a wavelength range between 50 nm and 1500 nm, preferably between 100 nm and 1200 nm, in particular between 200 nm and 1050 nm, in a particularly preferred manner between 275 nm and 400 nm.

8. Method according to any one of claims 1 to 7, **characterised in that** an excitation according to step c1) is carried out in a wavelength range between 50 nm and 600 nm, in particular between 100 nm and 500 nm, preferably between 150 nm and 400 nm.

9. Device (2) configured for detecting foreign substances, in particular harmful substances, in water, in particular in drinking water, preferably for carrying out a method according to any one of claims 1-8, comprising a preparation device (2) for preparing (8) a sample which has been removed from the water (M), wherein the preparation device (2) comprises a distillation device (7, 8, 9, 10, 11) for at least partially distilling the sample removed,
an analysis device (3) for analysing the condensate obtained by means of UV/VIS spectroscopy and/or fluorescence spectroscopy, wherein the analysis device (3) comprises at least one light source (13), in particular a laser, for exciting the condensate with light at at least one excitation wavelength and a detector (16) for recording at least one absorption spectrum and/or fluorescence spectrum of the light transmitted by the condensate and/or the light emitted by the condensate, and
an establishment device (4) for establishing at least one of the foreign substances in the condensate (M) using at least one of the recorded absorption and/or fluorescence spectrums.

10. Device according to claim 9, **characterised in that** a removal device (5) is arranged for removing a sample from the water (M) which is intended to be examined.

11. Device according to either claim 9 or 10, **characterised in that** an adjuvant addition device (6) is arranged for acidifying, alkalising, neutralising the sample and/or for adding complexing agents and/or for derivatisation.

12. Device according to any one of claims 9 to 11, **characterised in that** the analysis device (3) comprises an optical measurement cell (14) which is constructed in the form of an optical waveguide.

13. Device according to any one of claims 9 to 12, **characterised in that** the optical measurement cell (14) is produced from quartz and/or high-grade steel.

14. Device according to any one of claims 9 to 13, **characterised in that** a control device (17) is arranged to control the device.

## Revendications

1. Procédé destiné à détecter des substances étrangères, en particulier des polluants, dans l'eau, en particulier dans l'eau potable, approprié de préférence pour la réalisation avec un dispositif selon l'un des revendications 9-14, comprenant les étapes suivantes :
a) prélèvement (S1) d'un échantillon (M),
b) préparation (S2) de l'échantillon (M) prélevé, la préparation de l'échantillon comprenant une distillation (2) au moins partielle de l'échantillon (M) prélevé,
c) analyse du condensat obtenu au moyen de la spectroscopie UV/VIS et/ou de la spectroscopie de fluorescence, l'analyse comprenant les étapes suivantes :
c1) excitation (S4) du condensat avec de la lumière (13), en particulier d'un laser ou d'une lampe au deutérium, avec au moins une longueur d'onde d'excitation, et
c2) enregistrement (S5), au moyen d'un détecteur (16), d'au moins un spectre d'absorption et/ou d'un spectre de fluorescence de la lumière transmise par le condensat et/ou de la lumière émise par le condensat,
et
d) détermination (S6) d'au moins une des substances étrangères dans le condensat (M) analysé à l'aide d'au moins un des spectres d'absorption et/ou de fluorescence enregistrés.

2. Procédé selon la revendication 1, **caractérisé en ce que** la détermination (S6) selon l'étape d) comprend l'utilisation de méthodes d'étalonnage multivariées, méthodes chimiométriques, et/ou une comparaison des spectres enregistrés avec des spectres de référence.

3. Procédé selon l'une des revendications 1 à 2, **caractérisé en ce que** l'étalonnage multivarié est effectué au moyen de la régression des moindres carrés partiels.

4. Procédé selon l'une des revendications 1 à 3, **caractérisé en ce que** l'échantillon prélevé est acidifié, en particulier au moyen d'acide citrique et/ou d'acide sulfurique, alcalinisé, en particulier au moyen d'hydroxyde de sodium et/ou de soude, et/ou neutralisé.

5. Procédé selon l'une des revendications 1 à 4, **caractérisé en ce que** des agents complexants et/ou des moyens de dérivatisation sont ajoutés à l'échantillon prélevé.

6. Procédé selon l'une des revendications 1 à 5, **caractérisé en ce que** la distillation (2) est réalisée selon l'étape b) sous forme de distillation sous vide ou de distillation sous pression positive.

7. Procédé selon l'une des revendications 1 à 6, **caractérisé en ce que** le spectre selon l'étape c2) est enregistré dans une plage de longueur d'onde entre 50 nm et 1 500 nm, de préférence entre 100 nm et 1 200 nm, en particulier entre 200 nm et 1 050 nm, de façon particulièrement préférée entre 275 nm et 400 nm.

8. Procédé selon l'une des revendications 1 à 7, **caractérisé en ce qu'**une excitation selon l'étape c1) s'effectue dans une plage de longueur d'onde entre 50 nm et 600 nm, en particulier entre 100 nm et 500 nm, de préférence entre 150 nm et 400 nm.

9. Dispositif, équipé pour la détection de substances étrangères, en particulier des polluants, dans l'eau, en particulier dans l'eau potable, de préférence destiné à la réalisation d'un procédé selon l'une des revendications 1 à 8, comprenant un système de préparation (2) destiné à la préparation (8) d'un échantillon prélevé dans l'eau (M), le système de préparation (2) comprenant un système de distillation (7, 8, 9, 10, 11) destiné à la distillation au moins partielle de l'échantillon prélevé,
un système d'analyse (3) destiné à l'analyse du condensat obtenu au moyen de la spectroscopie UV/VIS et/ou de la spectroscopie de fluorescence, le système d'analyse (3) comprenant au moins une source de lumière (13), en particulier un laser, destinée à l'excitation du condensat par la lumière avec au moins une longueur d'onde d'excitation, et un détecteur (16) destiné à l'enregistrement d'au moins un spectre d'absorption et/ou d'un spectre de fluorescence de la lumière transmise par le condensat et/ou de la lumière émise par le condensat, ainsi
qu'un système de détermination (4) destiné à la détermination d'au moins une des substances étrangères dans le condensat (M) à l'aide d'au moins un des spectres d'absorption et/ou de fluorescence enregistrés.

10. Dispositif selon la revendication 9, **caractérisé en ce qu'**il est disposé un système de prélèvement (5) destiné au prélèvement d'un échantillon dans l'eau (M) à examiner.

11. Dispositif selon l'une des revendications 9 à 10, **caractérisé en ce qu'**il est disposé un système d'addition d'adjuvants destiné à l'acidification, l'alcalinisation, la neutralisation de l'échantillon et/ou destiné à l'ajout d'agents complexants et/ou destiné à la dérivatisation.

12. Dispositif selon l'une des revendications 9 à 11, **caractérisé en ce que** le système d'analyse (3) comprend une cellule de mesure optique (14) qui est constituée sous la forme d'un guide d'ondes optique.

13. Dispositif selon l'une des revendications 9 à 12, **caractérisé en ce que** la cellule de mesure optique (14) est réalisée en quartz et/ou en acier fin.

14. Dispositif selon l'une des revendications 9 à 13, **caractérisé en ce qu'**il est disposé un système de commande (17) destiné à commander le dispositif.
